# DEMANDE DE BREVET EUROPEEN

(11) **EP 4 760 347 A1**
(43) Date de publication de la demande: **17.06.2026**
(21) Numéro de dépôt: 25307036.1
(22) Date de dépôt: 05.12.2025
(51) Int. Cl.: G01S 7/52, G01S 15/89, A61B 8/00

(54) **DISPOSITIF ET PROCÉDÉ D'IMAGERIE ULTRASONORE 3D AVEC UN RÉSEAU DE TRANSDUCTEURS REPROGRAMMABLES**

(30) Priorité: 16.12.2024 FR 2414266
(71) Demandeur: VERMON SA, 37000 Tours (FR); Institut National de la Santé et de la Recherche Médicale, 75013 Paris (FR); Centre National de la Recherche Scientifique, 75016 Paris (FR); Ecole Supérieure de Physique et de Chimie Industrielles de la Ville de Paris, 75005 Paris (FR)
(72) Inventeur: GUERIF, Benjamin, 37000 TOURS (FR); PAPADACCI, Clément, 75015 PARIS (FR); DEFFIEUX, Thomas, 75015 PARIS (FR); TANTER, Mickael, 75015 PARIS (FR); PERNOT, Mathieu, 75015 PARIS (FR)
(74) Mandataire: Plasseraud IP

(57) **Abrégé**

Un appareil d'imagerie ultrasonore tridimensionnelle (1) d'une région d'intérêt d'un être vivant comprend:
- une sonde d'imagerie ultrasonore (100) comportant une pluralité de transducteurs ultrasonores élémentaires (102) répartis en un ensemble de groupes de transducteurs élémentaires (107), un circuit électronique d'émission (120) et un circuit électronique de réception (130) reliés aux transducteurs élémentaires (102);
- un système de contrôle (140) communiquant avec la sonde (100);
- ledit système de contrôle (140) étant configuré pour programmer pour chaque tir :
(a) le circuit électronique d'émission (120) de manière à adresser un ensemble de transducteurs élémentaires pour l'émission d'une onde ultrasonore non focalisée, et
(b) le circuit électronique de réception (130) de manière à adresser l'ensemble de groupes de transducteurs élémentaires ou une distribution de transducteurs élémentaires pour former des sous-ouvertures de réception apte à converger les ondes rétrodiffusées en un point focal situé derrière la sonde, du côté opposé à la région d'intérêt à imager.

## Description

### Domaine technique

La présente invention concerne un dispositif et un procédé d'imagerie ultrasonore tridimensionnelle 3D, en particulier un dispositif et un procédé d'imagerie ultrasonore 3D avec un réseau de transducteurs reprogrammables permettant une imagerie ultrarapide, avec un champ de vue large et profond tout en proposant une sonde ergonomique et compacte.

### Technique antérieure

Les systèmes d'imagerie médicale par ultrasons se sont développés très rapidement en raison de ses nombreux bénéfices au profit du diagnostic médical.

Généralement, les ultrasons peuvent être générés à partir d'une sonde comprenant un réseau d'une pluralité de transducteurs capables de générer individuellement des ondes ultrasonores. La sonde ultrasonore est adaptée pour être tenue par exemple par un praticien et déplacée sur différentes parties de l'anatomie d'un patient afin d'obtenir l'image de la région d'intérêt. Les transducteurs sont connectés à un système de contrôle ou système échographique par un câble. Les ondes ultrasonores peuvent être dirigées vers un milieu qui peut générer en réponse des signaux rétrodiffusés, qui à leur tour peuvent être enregistrés par le même réseau de transducteurs ou un réseau différent. Les signaux rétrodiffusés sont reçus et traités par un système de contrôle qui permet de reconstruire les signaux reçus sous la forme d'une image 2D ou 3D. Ces images 2D ou 3D peuvent être affichées en temps réel sur un écran du système de contrôle, permettant ainsi à un praticien de réaliser un diagnostic.

En imagerie conventionnelle tridimensionnelle, similaire à l'imagerie ultrasonore bidimensionnelle, l'image 3D peut être générée par balayage de la région volumétrique en focalisant successivement une pluralité de faisceaux ultrasonores puis en reconstruisant chacune des lignes correspondantes sur un volume. Lors de l'émission, les transducteurs individuels dans le réseau sont activés selon des retards afin de former un ou plusieurs faisceaux ultrasonores. Lors de la réception, les signaux reçus par les transducteurs sont retardés et sommés lors d'une phase de formation de faisceau pour reconstruire une ligne de l'image. Chaque transducteur est connecté à un canal d'un formateur de faisceau. Chacun des formateurs applique des retards aux signaux provenant du transducteur correspondant de manière à orienter et focaliser le faisceau formé par le formateur. Les signaux retardés par chaque formateur sont combinés pour former un faisceau orienté et focalisé. Les multiples faisceaux produits par des formateurs de faisceau fonctionnant en parallèle sont utilisés pour former plusieurs lignes d'une image ultrasonore. En d'autres termes chaque transducteur est associé à un canal sur lequel les signaux reçus sont transmis via un câble vers le système de contrôle. Les signaux peuvent être conditionnés dans l'électronique de sommation. Cette approche d'associer une électronique de sommation par transducteur devient complexe et n'est pas réalisable dans le cas d'une imagerie ultrasonore 3D sur une large volume. En effet, la capacité à imager un grand volume 3D est directement liée à la dimension du réseau de transducteurs. Afin de ne pas perdre en résolution spatiale, il est nécessaire d'utiliser un réseau de transducteurs dans lequel le pas entre deux éléments de transducteurs doit être inférieur à la longueur d'onde de l'onde ultrasonore afin de réduire les lobes secondaires et les lobes de réseau qui peuvent avoir un impact sur la qualité de l'imagerie. En outre, l'imagerie d'un large volume, c'est-à-dire avec un champ de vue et une profondeur plus importants, implique une large ouverture. Cette exigence conduit à des configurations des sondes ultrasonores comportant des milliers de transducteurs, nécessitant l'utilisation des milliers de canaux et un système d'imagerie plus complexe qui nécessiterait l'utilisation d'un gros câble pour transporter des milliers de canaux. La gestion de plusieurs milliers d'éléments pilotés par des milliers de voies électroniques entraîne des problèmes en termes de coût, de complexité de câblage et du système d'imagerie utilisé, rendant cette approche difficilement envisageable pour une application clinique.

En imagerie conventionnelle qui est basée sur la focalisation d'ondes ultrasonores par l'application des traitements de retards et sommation aux transducteurs ultrasonores pour obtenir la focalisation recherchée à l'émission et à la réception, une solution connue consiste à réduire le nombre de voies de transmission entre la sonde et le système de contrôle en intégrant directement au niveau du réseau matriciel 2D un circuit de traitement intégré spécifique à une application (ASIC) qui permet de réaliser une première étape de formation de faisceaux au niveau de la sonde par l'application des traitements de retards et sommation par groupes de transducteurs qui sont aussi appelés sous-ouvertures de réception, permettant d'obtenir un signal électrique par groupe de transducteurs au lieu d' un signal électrique par transducteur. Dans une seconde étape, les signaux électriques générés par les circuits de micro-formation de faisceaux sont transmis au système de contrôle pour la reconstruction d'une ligne de l'image 3D en appliquant de nouveau des traitements de retard et sommation. En d'autres termes, la reconstruction d'une ligne de l'image 3D est décomposée en deux étapes, une première étape au niveau de la sonde et une seconde étape au nveau du système de contrôle. Cette solution permet de piloter un réseau de plusieurs milliers de transducteurs pour imager un large volume avec un nombre réduit de voies adapté pour fonctionner avec un unique système d'imagerie. Toutefois, cette solution n'est pas adaptée pour des modalités d'imagerie ultrarapides avec une cadence d'imagerie très élevée de l'ordre de 1000 images par seconde qui sont développées pour observer des phénomènes très rapides, telles que l'échocardiographie, l'élastographie, la microscopie de localisation ultrarapide. En effet, dans l'imagerie ultrarapide, la reconstruction de l'image 3D utilise l'ensemble des signaux rétrodiffusés par le milieu et reçus par les éléments pour chaque tir pour la reconstruction de l'ensemble du volume de la région d'intérêt, ce qui n'est plus possible après l'étape de micro-formation des faisceaux.

Une autre solution consiste à utiliser des réseaux clairsemés ou « sparses » pour réduire le nombre de voies en transmission et réception. Cette solution consiste à adresser une partie des éléments parmi plusieurs milliers d'éléments en transmission et en réception de sorte que le nombre de signaux électriques à traiter soit compatible avec le nombre de voies d'échantillonnage que possède un système d'imagerie conventionnel. Bien qu'une telle sonde permette une imagerie ultrarapide 3D, le fait d'utiliser un nombre de transducteurs inférieur à une sonde dite complètement peuplée, fait apparaître des lobes secondaires et diminue de manière significative la qualité d'imagerie 3D. En outre, cette solution entraîne une réduction de la surface sensible utilisée en transmission et en réception car tous les éléments ne sont pas utilisés, entraînant une diminution du rapport signal à bruit et donc la qualité de l'image. Cette technique de réseau « sparse » n'est pas par exemple adaptée à l'imagerie à travers les os où l'on a besoin d'un maximum d'énergie transmise et de sensibilité de réception.

Par voie de conséquence, la capture de grands volumes 3D, à haute cadence d'imagerie reste un défi en microscopie de localisation ultrasonore (ULM), et plus particulièrement dans le domaine de l'imagerie transcrânienne.

La présente invention a notamment pour but de pallier ces inconvénients et de proposer un dispositif et un procédé d'imagerie permettant une imagerie ultrasonore 3D avec un large champ de vision, tout en maintenant la qualité d'imagerie, et adaptée pour des cadences d'imagerie élevées, supérieure à 1000 images par seconde, sans augmenter notablement la complexité et le coût des appareils d'imagerie acoustique sur lesquels il est mis en œuvre.

### Résumé de l'invention

Les modes de réalisation qui y sont décrits permettent d'améliorer les appareils et les procédés d'imagerie ultrasonore 3D.

A cette fin, selon un premier aspect, la présente divulgation concerne un appareil d'imagerie ultrasonore tridimensionnelle d'une région d'intérêt du corps d'un être vivant, ledit appareil comprenant :
- une sonde d'imagerie ultrasonore comportant une pluralité de transducteurs élémentaires agencés en matrice selon des rangées et des colonnes formant un réseau, lesdits transducteurs élémentaires étant répartis en un ensemble de groupes de transducteurs élémentaires, un circuit électronique d'émission et un circuit électronique de réception reliés aux transducteurs élémentaires;
- un système de contrôle configuré pour communiquer avec la sonde ;
- ledit circuit électronique de réception comprenant un circuit de micro-formation de faisceaux pour chaque groupe de transducteurs élémentaires, ledit circuit de micro-formation de faisceaux ayant une entrée reliée individuellement à chaque transducteur élémentaire du groupe et une sortie reliée au système de contrôle, ledit circuit de micro-formation de faisceaux étant adapté à fournir un unique signal généré à partir des signaux de réponse des transducteurs élémentaires du groupe ;
- ledit système de contrôle étant configuré pour programmer pour chaque tir :
   (a) le circuit électronique d'émission de manière à adresser un ensemble de transducteurs élémentaires adjacents parmi la pluralité de transducteurs élémentaires afin de former une ouverture d'émission et à appliquer une loi de retards aux transducteurs élémentaires dudit ensemble pour l'émission d'une onde ultrasonore non focalisée;
   (b) chaque circuit électronique de micro-formation de faisceaux de manière à adresser le groupe de transducteurs élémentaires associé ou un transducteur élémentaire du groupe afin de former une sous-ouverture de réception associée à chaque groupe ayant un point de focalisation situé derrière le réseau, du côté opposé à la région d'intérêt à imager, adaptée pour la réception des ondes rétrodiffusées, de sorte qu'une pluralité de sous-ouvertures de réception est formée.

Dans la présente invention, le réseau de transducteurs élémentaires est divisé en un ensemble de groupes, chaque groupe étant associé à un circuit de micro-formation de faisceaux dédié. Lors de la réception, chaque circuit de micro-formation de faisceaux forme une sous-ouverture de réception à partir des transducteurs du groupe associé, et l'ensemble de ces sous-ouvertures fonctionne en parallèle. Le nombre de sous-ouvertures de réception formées est égal au nombre de groupes de transducteurs. Ainsi, l'application du retard sur les transducteurs élémentaires au sein de chaque groupe permet d'obtenir un réseau épars de sous-ouvertures de réception ayant chacune un point de focalisation derrière le réseau, du côté opposé à la région d'intérêt à imager. La formation simultanée de plusieurs sous-ouvertures de réception, chacune focalisée virtuellement derrière le réseau, permet d'augmenter la zone de réception, de réduire les lobes de réseau et d'améliorer la qualité de l'image 3D, tout en limitant la complexité électronique et le nombre de voies de transmission. Cette architecture où chaque groupe de transducteurs forme une sous-ouverture de réception distincte conduit à un nombre total de sous-ouvertures correspondant au nombre de groupes. En d'autres termes, à chaque tir, en réception, l'ensemble des groupes de transducteurs de la sonde est adressé par le système de contrôle pour former une pluralité de sou-ouvertures de réception

En outre, le système de contrôle de l'invention est configuré pour reprogrammer, à chaque tir, l'ouverture d'émission et la configuration des sous-ouvertures de réception qui est différente de la configuration de l'ouverture d'émission. Cette flexibilité permet d'adapter dynamiquement la géométrie et la focalisation en fonction des besoins d'imagerie.

Les caractéristiques exposées dans les paragraphes suivants peuvent, optionnellement, être mises en œuvre, indépendamment les unes des autres ou en combinaison les unes avec les autres :

Le système de contrôle peut être configuré pour reprogrammer le circuit électronique d'émission pour modifier l'ouverture d'émission et les délais d'émission pour l'émission d'une onde ultrasonore non focalisée à chaque tir.

Le système de contrôle peut être configuré pour reprogrammer les circuits électroniques de micro-formations de faisceaux pour modifier les sous-ouvertures de réception et les délais de réception pour la réception des ondes rétrodiffusées à chaque tir.

Le système de contrôle peut être configuré pour programmer le circuit électronique d'émission pour appliquer des retards d'émission sur les transducteurs de sorte que l'onde ultrasonore non focalisée émise est une onde plane ou divergente.

Chaque circuit de micro-formation de faisceaux peut comprendre un circuit de réception et à retard pour chaque transducteur du groupe et un circuit de micro-sommation des signaux.

De préférence, les circuits de réception et à retard du groupe sont aptes à appliquer une loi de retards sur les transducteurs élémentaires du groupe de manière à former une lentille virtuelle dont le point de focalisation est situé derrière le réseau, du côté opposé à la région à imager.

Ainsi, lors de la réception, une pluralité de lentilles virtuelles est formée. Le nombre de lentilles virtuelles est égal au nombre de groupes de transducteurs du réseau.

En outre, le système de contrôle peut être configuré pour programmer les circuits de réception et à retard de sorte que les lentilles virtuelles formées ont des distances focales identiques.

Selon une variante, le système de contrôle peut être configuré pour programmer les circuits de réception et à retard de sorte qu'au moins deux lentilles virtuelles ont des distances focales différentes.

Selon encore une autre variante, le système de contrôle est configuré pour programmer les circuits de réception et à retard de sorte qu'au moins deux lentilles virtuelles ont des directions de focalisation différentes.

Selon la présente divulgation, le système de contrôle peut être configuré pour programmer les circuits de réception et à retard de sorte qu'une pluralité de lentilles virtuelles ont des points de focalisation distribués de manière aléatoire derrière le réseau de transducteurs, du côté opposé à la région d'intérêt à imager. Ainsi, de manière avantageuse, ces combinaisons d'agencements aléatoires des points de focalisation des lentilles virtuelles perturbent la périodicité du système, ce qui permet d'améliore la qualité d'image en imagerie ultrasonore volumique 3D.

Selon un mode de réalisation, le circuit de réception et à retard comprend un amplificateur de réception, un circuit d'ajustement temporel de gain et un circuit à retard.

Selon un autre aspect, la divulgation concerne un procédé d'imagerie ultrasonore tridimensionnelle (3D) d'une région d'intérêt du corps d'un être vivant à l'aide d'un appareil d'imagerie ultrasonore tridimensionnelle tel que défini ci-dessus, ledit procédé comprenant pour chaque tir:
- (a) une étape de programmation au cours de laquelle le circuit électronique d'émission est programmé (PROGRAM_EMI) pour adresser un ensemble de transducteurs élémentaires adjacents parmi la pluralité de transducteurs élémentaires afin de former une ouverture d'émission et à appliquer une loi de retards adaptée aux transducteurs élémentaires dudit ensemble pour l'émission d'une onde ultrasonore non focalisée ;
- (b) une étape de programmation au cours de laquelle chaque circuit électronique de micro-formation de faisceaux est programmé pour adresser le groupe de transducteurs élémentaires associé ou au moins un transducteur élémentaire du groupe afin de former une sous-ouverture de réception ayant un point de focalisation situé derrière le réseau, du côté opposé à la région d'intérêt à imager, adaptée pour la réception des ondes rétrodiffusés (PROGRAM_REC), de sorte qu'une pluralité de sous-ouvertures de réception est formée;
- (c) une étape d'émission au cours de laquelle l'onde ultrasonore non focalisée est émise en direction de la région d'intérêt par ladite ouverture d'émission formée par ledit ensemble de transducteurs élémentaires adjacents adressés (EMI_US) ;
- (d) une étape de réception au cours de laquelle les ondes rétrodiffusées provenant de la région d'intérêt sont reçues par chaque sous-ouverture non focalisée formée dans la sonde (REC_US);
- (e) une étape de transmission de signaux au cours de laquelle le signal fourni par chaque circuit de micro-formation de faisceaux est transmis vers le système de contrôle (TRANS_SIG);
- (f) une étape de construction d'image 3D au cours de laquelle les signaux sont retardés et sommés dans le système de contrôle (140) pour construire une image volumétriques ultrasonore 3D correspondant à l'onde émise (CONST_IMG_COM).

Le procédé peut en outre comprendre l'une et/ou l'autre des caractéristiques suivantes :
Les étapes (a) à (f) peuvent être répétées pour émettre une série d'ondes ultrasonore non focalisée selon différentes directions de propagation et pour acquérir une série de signaux pour chaque onde émise, lesdits signaux permettant de former une série d'images volumétriques 3D et dans lequel le procédé comprend en outre une étape de construction d'une image composée au cours de laquelle lesdites images volumétriques 3D sont sommées de manière cohérente pour former une image composée finale.

Le système de contrôle peut reprogrammer le circuit d'émission pour modifier l'ouverture d'émission et les délais d'émission pour l'émission d'une onde ultrasonore non focalisée à chaque tir.

Le système de contrôle peut reprogrammer le circuit électronique de micro-formation de faisceaux pour modifier les sous-ouvertures de réception et les délais de réception pour la réception des ondes rétrodiffusées à chaque tir.

### Brève description des dessins

D'autres caractéristiques, détails et avantages apparaîtront à la lecture de la description détaillée ci-après, et à l'analyse des dessins annexés, sur lesquels :
**Fig. 1**

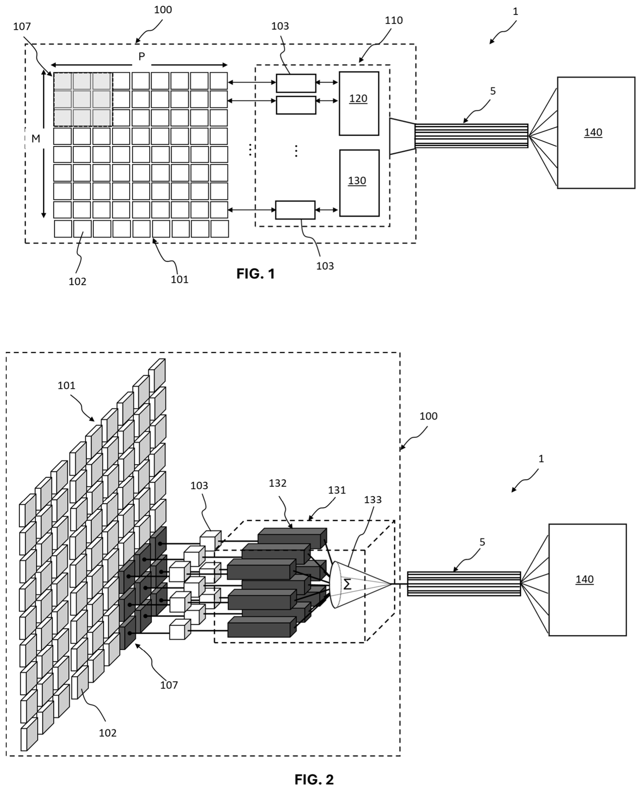

   [Fig. 1] La figure 1 représente un schéma fonctionnel d'un appareil d'imagerie ultrasonore 3D selon un mode de réalisation ;
**Fig. 2**
   [Fig. 2] La figure 2 représente plus détail le circuit de micro-formation de faisceaux de l'appareil de la figure 1 ;
**Fig. 3**

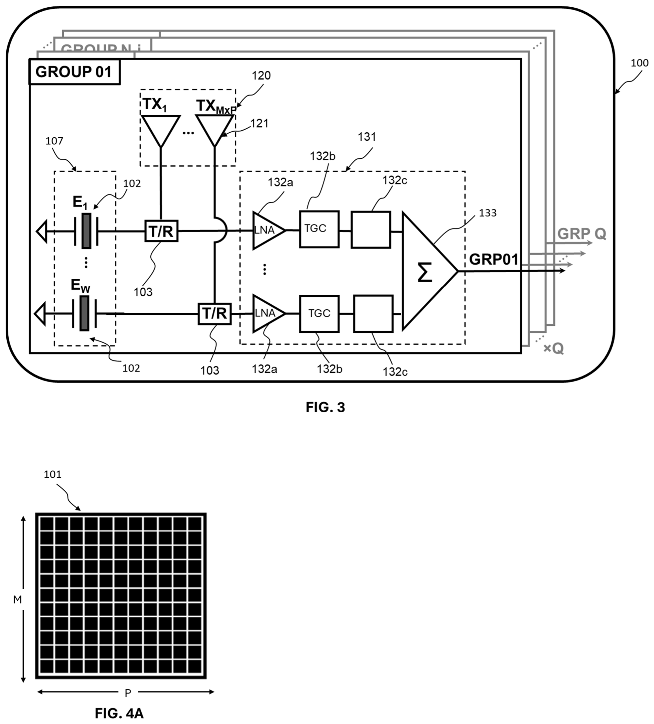

   [Fig. 3] La figure 3 représente plus détail le circuit élémentaire de réception et à retard de la figure 2 ;
**Fig. 4A**
   La figure 4A représente une vue schématique de dessus des transducteurs de la sonde de la figure 1 dans une étape d'émission dans laquelle l'ensemble des transducteurs sont utilisés pour émettre une onde ultrasonore de manière à former une ouverture d'émission ;
**Fig. 4B**

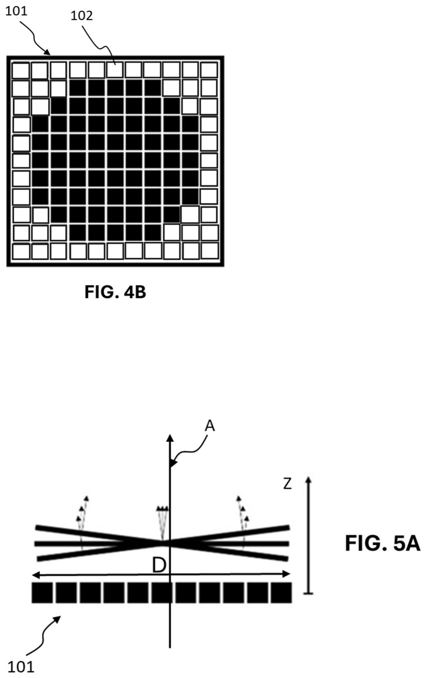

   [Fig. 4B] La figure 4B représente une vue schématique de dessus d'un réseau de transducteurs de la sonde de la figure 1 dans une étape d'émission dans laquelle une partie des transducteurs périphériques ne sont pas utilisés de manière à former une ouverture d'émission dense mais avec une autre forme géométrique que celle de l'ouverture d'émission de la figure 4A;
**Fig. 5A**
   [Fig. 5A] La figure 5A représente une vue schématique de côté des transducteurs de la sonde de la figure 4A dans une étape d'émission d'une onde plane ;
**Fig. 5B**

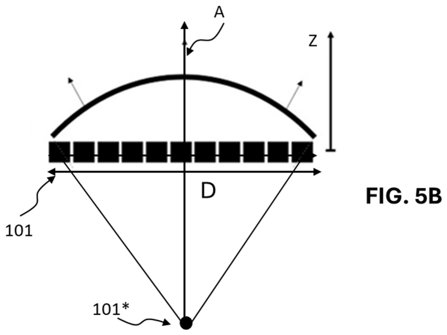

   [Fig. 5B] La figure 5B représente une vue schématique de côté des transducteurs de la sonde de la figure 4A dans une étape d'émission d'une onde divergente ;
**Fig. 5C**

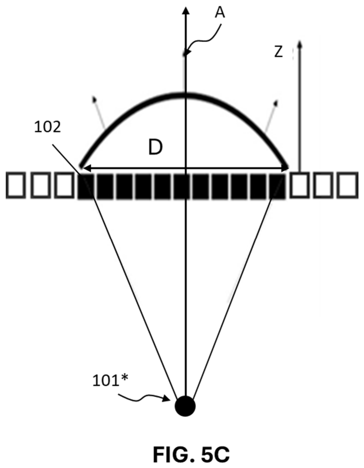

   [Fig. 5C] La figure 5C représente une vue schématique de côté des transducteurs de la sonde de la figure 4B dans une étape d'émission d'une onde divergente ;
**Fig. 6A**

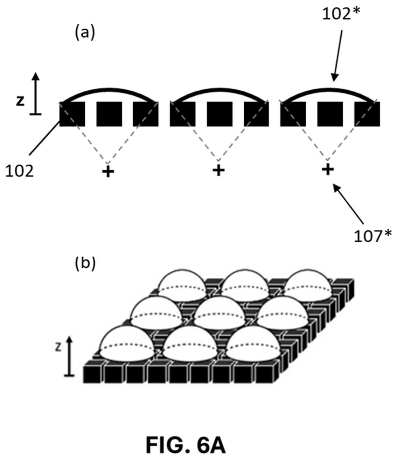

   [Fig. 6A] La figure 6A représente une vue schématique des transducteurs élémentaires de la sonde de la figure 2 dans une étape de réception dans laquelle le circuit de micro-formation de faisceaux est programmé par le système de contrôle de sorte que chaque sous-ensemble de transducteurs élémentaires émule une lentille acoustique virtuelle pour faire converger une onde acoustique en phase de réception en un foyer focal virtuel situé à l'arrière du réseau, du côté opposé au milieu extérieur à imager, le rayon de courbure et l'orientation de toutes les lentilles virtuelles convergentes étant identiques ;
**Fig. 6B**

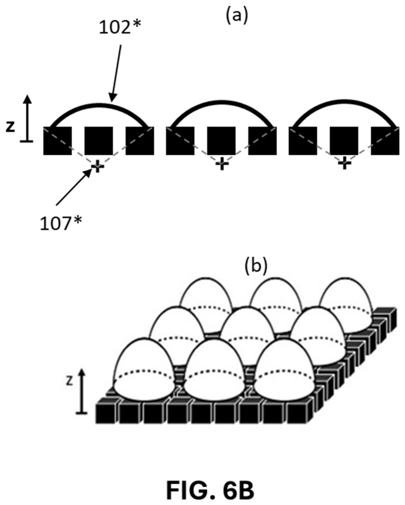

   [Fig. 6B] La figure 6B représente une vue schématique des transducteurs élémentaires de la sonde de la figure 2 dans une étape de réception dans laquelle le circuit de micro-formation de faisceaux est programmé par le système de contrôle de sorte que les sous-ensembles de transducteurs émulent un réseau de lentilles virtuelles convergentes, configurées pour faire converger une onde acoustique en phase de réception en un foyer focal virtuel situé à l'arrière du réseau, du côté opposé au milieu extérieur à imager en réception avec un rayon de courbure plus important que celui de la figure 6A, le rayon de courbure et l'orientation de toutes les lentilles virtuelles convergentes étant identiques ;
**Fig. 6C**

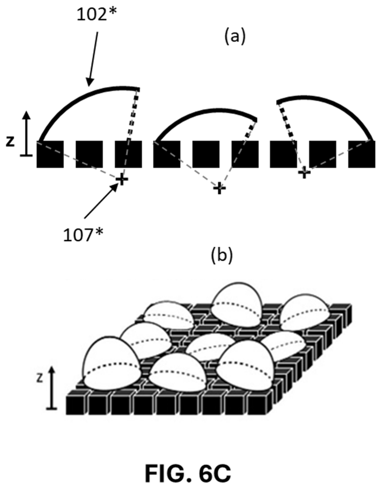

   [Fig. 6C] La figure 6C représente une vue schématique des transducteurs de la sonde de la figure 2 dans une étape de réception dans laquelle le circuit de micro-formation de faisceaux est programmé par le système de contrôle de sorte que les sous-ensembles de transducteurs forment un réseau de lentilles virtuelles convergentes, configurées pour faire converger une onde acoustique en phase de réception en un foyer focal virtuel situé à l'arrière du réseau, du côté opposé au milieu extérieur à imager en réception le rayon de courbure et l'orientation des lentilles virtuelles variant d'une lentille à l'autre de manière pseudo-aléatoire prédéfini selon un algorithme ;
**Fig. 7A**

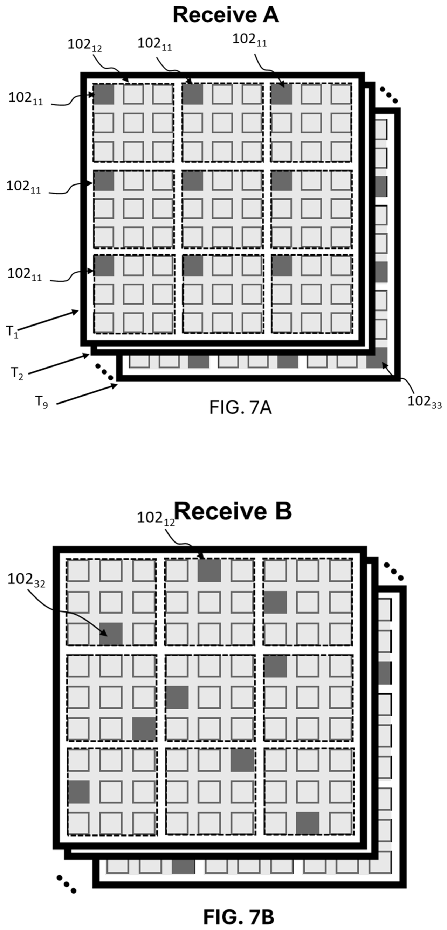

   [Fig. 7A] La figure 7A représente une vue schématique de dessus du réseau 2D de la figure 2 dans une étape de réception dans laquelle le circuit de micro-formation de faisceaux de chaque groupe est programmé par le système de contrôlé pour adresser un transducteur de chaque groupe de transducteurs élémentaires, le motif d'adressage étant identique pour tous les groupes du réseau et identique pour chaque tir ;
**Fig. 7B**
   [Fig. 7B] La figure 7B représente une vue schématique de dessus du réseau 2D de la figure 2 dans une étape de réception dans laquelle le circuit de micro-formation de faisceaux de chaque groupe est programmé par le système de contrôlé pour adresser un transducteur de chaque groupe de transducteurs élémentaires, le motif d'adressage étant différent d'un groupe à l'autre et différent pour chaque tir ;
**Fig. 8**

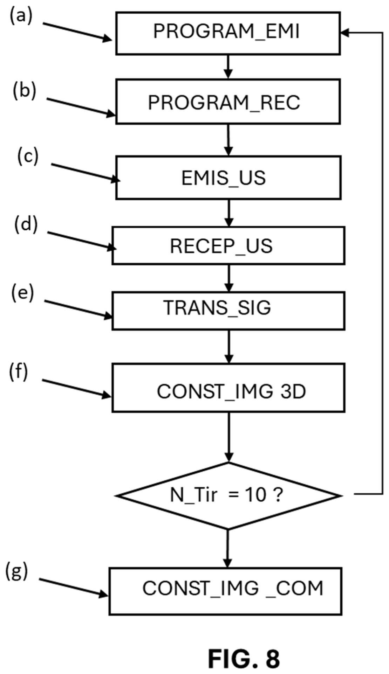

   [Fig. 8] La figure 8 représente les principales étapes d'un procédé d'imagerie ultrarapide mettant en œuvre l'appareil de la figure 3 dans le cas où les groupes de transducteurs élémentaires sont contrôlés individuellement pour former des sous-ouvertures de réception non focalisées ou émuler des lentilles virtuelles divergentes.

### Description des modes de réalisation

### Définitions

Dans les figures, les mêmes références désignent les éléments identiques ou similaires.

Dans la présente divulgation, le terme « ouverture » tel qu'utilisé ici désigne une ouverture disponible et formée par un ensemble des transducteurs disposés dans un réseau bidimensionnel, l'ouverture étant utilisée pour émettre des ondes ultrasonores et/ou recevoir des signaux rétrodiffusés.

Dans la présente divulgation, le terme « sous-ouverture » tel qu'utilisé ici désigne une ouverture formée par un groupe de transducteurs, une sous-ouverture d'émission étant formée par un sous-ensemble de transducteurs élémentaires du réseau bidimensionnel lors de la phase d'émission d'une onde ultrasonore, une sous-ouverture de réception étant formée par un sous-ensemble de transducteurs élémentaires lors de la phase de réception des signaux rétrodiffusés par le milieu.

Dans la présente divulgation, le terme « sous-ouverture dense » tel qu'utilisé ici désigne une ouverture formée par un groupe de transducteurs adjacents.

Dans la présente divulgation, on entend par « micro-formation de faisceaux » une stratégie de formation de faisceau type retard et somme décomposée en deux étapes. La première étape est réalisée dans la sonde ultrasonore et est réalisée analogiquement ou numériquement par un ensemble de circuits électroniques de micro-formation de faisceaux dédiés pour former chacun une sous-ouverture non focalisée. Les signaux ainsi microformés, c'est-à-dire retardés et sommés dans la sonde sont transmis depuis la sonde vers un système de contrôle externe à la sonde. La deuxième étape est réalisée dans le système de contrôle en appliquant de nouveau une stratégie de formation de faisceau par l'application de retards et de sommes entre les signaux microformés pour reconstruire une image 3D.

Dans la présente divulgation, le terme « sous-ouverture de réception non focalisée » tel qu'utilisé ici désigne un groupe de transducteurs élémentaires dont les signaux de réponse générés sont retardés et sommés de manière à émuler une lentille virtuelle dont le point de focalisation virtuel est situé derrière le réseau de transducteurs élémentaires du côté opposé à la région à imager.

Dans la présente divulgation, le terme « lentille virtuelle tel qu'utilisé ici désigne une lentille virtuelle acoustique ayant une forme courbée orientée vers le milieu extérieur à imager et apte à faire converger en phase de réception un faisceau d'ondes ultrasonores en un point focal situé derrière le réseau d'éléments transducteurs, du côté opposé à la région à imager.

La figure 1 est une vue schématique représentant un exemple d'appareil d'imagerie ultrasonore 1 configuré pour réaliser une image ultrasonore tridimensionnelle (3D) d'un milieu à imager par émission et réception d'ondes ultrasonores.

Le milieu à imager désigne par exemple une région d'intérêt du corps d'un patient ou d'un animal. A titre d'exemple, l'appareil présenté est adapté pour réaliser de l'imagerie ultrarapide par ultrasons tridimensionnelle 3D ou 4D (3D + temps) du cœur d'un être vivant.

L'appareil d'imagerie 1 peut comprendre une sonde 100 et un système de contrôle 140 connecté à la sonde par l'intermédiaire d'un câble 5.

La sonde 100 comprend un réseau bidimensionnel (2D) 101 de transducteurs ultrasonores élémentaires 102 et un circuit électronique de contrôle des transducteurs ultrasonores élémentaires 110. Le circuit électronique de contrôle 110 comprend un circuit électronique d'émission 120 adapté pour appliquer un signal électrique d'excitation aux transducteurs élémentaires pour l'émission d'une onde ultrasonore, un circuit électronique de réception 130 adapté à contrôler les transducteurs élémentaires pour la réception des ondes rétrodiffusées, une pluralité de commutateurs d'émission-réception 103 permettant de connecter les transducteurs ultrasonore élémentaires 102 soit au circuit d'émission 120 soit au circuit de réception 130.

Le système de contrôle 140 est configuré pour commander les circuits électroniques d'émission et de réception 120, 130 de manière à programmer l'activation ou l'extinction des transducteurs ultrasonores élémentaires de la sonde ultrasonore 100 pour modifier l'ouverture d'émission et l'ouverture de réception avant chaque tir, et pour enregistrer et traiter les signaux rétrodiffusés transmis par la sonde afin de générer une image tridimensionnelle du milieu d'intérêt.

Le système de contrôle 140 peut par exemple comprendre une unité de commande et un ordinateur. L'unité de commande est utilisée pour commander les circuits électroniques d'émission 120 et de réception 130, acquérir et traiter les signaux transmis par la sonde 100, tandis que l'ordinateur est utilisé pour commander l'unité de commande, et construire des images 3D à partir des signaux acquis et traités par l'unité de commande et déterminer des paramètres de quantification à partir des images 3D. Selon une variante et comme l'illustre la figure 1, un seul appareil électronique 140 peut remplir toutes les fonctionnalités de l'unité de contrôle et de l'ordinateur. Selon une autre variante, au moins une partie des fonctionnalités de l'unité de commande et de l'ordinateur peut être intégrée dans la sonde 100.

Le câble 5 permet de relier la sonde 100 au système de contrôle 140 pour la transmission des instructions de commande vers le circuit électronique d'émission 120 et le circuit électronique de réception 130 logés dans la sonde afin de commander individuellement les transducteurs élémentaires. Selon un exemple de réalisation, le câble 5 permet également de transmettre les signaux électriques acquis par la sonde 100 depuis la sonde 100 vers le système de contrôle 140. Selon une variante, une liaison sans-fils peut remplacer le câble 5.

L'appareil d'imagerie peut comporter en outre un écran ou tout autre interface utilisateur permettant de visualiser une image du champ d'observation.

La sonde 100 est par exemple une sonde portative pouvant être positionnée et/ou déplacé par le praticien sur le corps du patient ou de l'animal pour imager une région d'intérêt.

La sonde 100 peut comprend une pluralité de transducteurs ultrasonores élémentaires 102 agencés en matrice selon M rangées et P colonnes sous forme d'un réseau 101. Les transducteurs élémentaires sont par exemple sensiblement tous identiques. Le pas entre deux transducteurs est de l'ordre de la longueur d'onde dans la direction des rangées et dans la direction des colonnes. Un exemple d'une matrice 101 de 9*9 de transducteurs élémentaires est montré sur la Figure 1. En pratique, la matrice peut comprend plusieurs milliers à plusieurs dizaines de milliers transducteurs qui peuvent être adressés individuellement par le circuit électronique d'émission 120 et le circuit de réception 130 pour émettre une onde ultrasonore et recevoir une onde ultrasonore rétrodiffusée par le milieu insonifié. La sonde peut comprendre par exemple 1024 éléments de transducteur (32*32), avec un pas de 0,3 mm. Les transducteurs peuvent émettre par exemple à une fréquence centrale comprise entre 1 et 10 MHz, par exemple de 3 MHz.

Dans l'exemple de la figure 1, les transducteurs élémentaires ont une forme carrée et le pas inter-transducteur étant identique dans la direction des rangées et dans la direction des colonnes. Selon un autre exemple de réalisation, le pas inter-transducteur peut être différent dans les deux directions et les transducteurs peuvent avoir une forme rectangulaire. La description qui suit sera faite en prenant l'exemple de la figure 1, mais d'autres formes de réseau de transducteurs sont également possibles dans le cadre de la présente divulgation.

De manière connue, chaque transducteur élémentaire comprend deux électrodes permettant d'appliquer un signal électrique d'excitation au transducteur et de lire un signal électrique de réponse du transducteur. Le commutateur d'émission-réception 103 est configuré pour connecter une électrode du transducteur 102 de la matrice 101 soit à une borne de sortie du circuit électronique d'émission 120 pendant la phase d'émission d'une onde ultrasonore, soit à une borne d'entrée du circuit de réception 130 pendant la phase de réception d'une onde ultrasonore.

En fonctionnement, l'ensemble des transducteurs 102 est placé sur une région d'intérêt d'un patient dont on souhaite acquérir l'image 3D. Le circuit électronique d'émission 120 est configuré pour appliquer des impulsions aux transducteurs de façon à provoquer l'émission d'ondes ultrasonores en direction de la région d'intérêt. Les ondes ultrasonores émises par les transducteurs sont rétrodiffusées par le milieu insonifié et reçues par les transducteurs qui les convertissent en signaux électriques dits signaux RF bruts. L'étape d'émission et l'étape de réception forment un tir ou une étape de mesure. Les signaux RF bruts sont reçus par le circuit électronique de réception 130 au niveau de la sonde pour être préformés puis transmis au dispositif de commande et traitement 140 via le câble 5 aux fins de la reconstruction d'une image 3D à partir desdits signaux préformés.

Dans la présente divulgation, afin de pouvoir mettre en oeuvre une imagerie ultrarapide, le système d'imagerie 140 de la présente divulgation est configuré pour programmer le circuit électronique d'émission 120 de sorte que dans une étape d'émission les transducteurs élémentaires de la matrice soient activés pour former une ouverture d'émission adaptée à l'émission d'une onde non focalisée, c'est à dire une onde plane ou divergente pour insonifier (exciter acoustiquement) l'intégralité du milieu. Le signal capté par chaque transducteur provient de l'ensemble du milieu. En outre, pour maintenir la compacité de la sonde et limiter le nombre de voies de réception, le système de contrôle 140 est configuré pour programmer le circuit électronique de réception 130 de sorte que dans une étape de réception les transducteurs élémentaires de la matrice soient activés pour former des sous-ouvertures de réception adaptées à transmettre un nombre de signaux de réponse microformés compatible avec le nombre de voies d'échantillonnage disponibles sur le système de contrôle 140. Le système de contrôle 140 est configuré pour générer un volume ou une image 3D correspondant à l'onde transmise à partir des signaux de réponse microformés acquis en appliquant une loi des retards et de sommation.

Afin d'augmenter la qualité d'imagerie, le système de contrôle 140 est configuré pour programmer le circuit électronique d'émission 120 et le circuit électronique de réception 130 de manière à répéter l'étape d'émission et l'étape de réception à haute cadence pour émettre par le réseau de transducteur successivement une série de N ondes ultrasonore planes ou divergentes non focalisées ayant respectivement des directions de propagation différentes pour réaliser N tirs afin d'obtenir N acquisitions de signaux électriques. Les N acquisitions de signaux électriques permettent de former N images 3D ou N volumes qui sont ensuite sommées de manière cohérente pour former une image composée de haute qualité.

De manière avantageuse, le système de contrôle 140 est configuré pour reprogrammer le circuit électronique d'émission 120 et le circuit électronique de réception 130 entre deux étapes de mesure ou deux tirs de manière à modifier respectivement l'ouverture d'émission et les sous-ouvertures de réception formées par des groupes de transducteurs ou l'ouverture de réception formée par une distribution éparse de transducteurs répartis parmi l'ensemble des transducteurs du réseau afin d'augmenter la sensibilité d'imagerie.

Le système de contrôle 140 est configuré pour ensuite sommer de manière cohérente les N images 3D angulées ou les N volumes obtenus après chaque tir afin de générer une image 3D finale de haute qualité de la région.

En référence à la figure 3, le circuit d'émission 120 comprend pour chaque transducteur un circuit élémentaire d'émission 121.

Lors de la phase d'émission, le commutateur d'émission-réception 103 connecte une électrode du transducteur élémentaire à une borne de sortie du circuit élémentaire d'émission. Un signal électrique d'excitation est appliqué au transducteur élémentaire par le circuit d'émission élémentaire. Sous l'effet de l'impulsion électronique, chacun des transducteurs élémentaires génère une onde acoustique sphérique qui se propage devant lui, et qui s'ajoute à la contribution de celles de ses voisins. Le système de contrôle 140 est configuré pour commander indépendamment chaque transducteur élémentaire via le circuit d'émission élémentaire pour ajouter un retard particulier de sorte que les ondes sphériques émises par chacun des transducteurs élémentaires se superposent pour former un front d'onde particulier, par exemple une onde ultrasonore non focalisée, par exemple plane ou une onde divergente afin d'insonifier l'ensemble du milieu.

Selon un autre mode de réalisation, le système de contrôle 140 est configuré pour programmer les circuits d'émission élémentaires 121 de manière à piloter indépendamment chaque transducteur élémentaire pour choisir les transducteurs à activer afin obtenir une ouverture d'émission dense particulière avec une géométrie particulière. Cette activation peut être réalisée par exemple en augmentant ou en diminuant l'amplitude du signal d'excitation transmis au transducteur correspondant, voire en imposant une amplitude nulle, ce qui correspond à l'extinction de la voie associée au transducteur élémentaire. Une ouverture d'émission dense désigne la configuration où l'ouverture est obtenue en utilisant un groupe de transducteurs adjacents de la matrice de la sonde. Selon un autre mode de réalisation, le dispositif de commande et traitement 140 est configuré pour programmer les circuits d'émission élémentaires 121 de sorte que l'ensemble des transducteurs de la matrice soient utilisés pour former l'ouverture d'émission.

De manière avantageuse, le système de contrôle 140 est également configuré pour programmer les circuits d'émission élémentaires 121 de manière à appliquer des délais d'émission sur les transducteurs pour ajuster le rayon de courbure de l'onde divergente et/ou la direction de propagation de l'onde plane ou divergente.

La figure 4A montre un exemple où l'ouverture d'émission est obtenue en utilisant ou adressant l'ensemble des transducteurs du réseau dans un état d'émission, permettant d'obtenir une puissance de l'onde ultrasonore plus élevée. Sur la figure 4A, les carrés sombres représentent les transducteurs activés ou utilisés pour émettre une onde plane ou sphérique.

La figure 4B montre un exemple où les transducteurs situés à la périphérie du réseau ne sont pas utilisés, permettant ainsi de former une ouverture d'émission dense avec une géométrie différente à celle de la figure 4A. Sur la figure 4B, les carrés blancs représentent les transducteurs élémentaires non activés et les carrés sombres représentent les transducteurs élémentaires activés pour émettre une onde plane ou sphérique et qui contribuent donc à la formation de l'onde non focalisée. Les transducteurs non utilisés peuvent correspondre à des transducteurs dont les voies associées sont éteintes ou à des transducteurs dont l'amplitude du signal d'excitation transmis est pondérée. De manière particulièrement avantageuse, l'amplitude du signal est diminuée pour les transducteurs en périphérie de l'ouverture d'émission dense de manière à réduire les effets de bord de l'onde émise.

Les figures 5A-5C illustrent une vue schématique de côté du réseau de la figure 1 avec des ouvertures d'émission différentes et des délais d'émission différents.

Selon un mode de réalisation et en référence à la figure 5A, les circuits d'émission élémentaires sont programmés par le système de contrôle 140 pour activer l'ensemble des transducteurs élémentaires de la matrice, à savoir les 9*9 transducteurs élémentaires avec une ouverture d'émission maximale (D) et pour ajouter un délai d'émission sur les transducteurs élémentaires de manière à émettre une onde ultrasonore plane selon différentes directions de propagation par rapport à l'axe acoustique du réseau A. Lorsque les circuits d'émission élémentaires transmettent une impulsion électronique sans déphasage aux transducteurs du réseau, l'onde plane est émise dans la direction de l'axe A. L'ensemble du milieu est donc insonifié en un seul tir et tout le milieu rétrodiffuse l'onde ultrasonore.

Afin d'augmenter la qualité d'imagerie, les circuits d'émission élémentaires sont programmés par le système de contrôle 140 de sorte que l'onde plane est émise par le réseau selon plusieurs directions faisant un angle avec l'axe acoustique A pour incliner le front d'onde. Ainsi, le milieu est insonifié avec une succession d'ondes planes angulées, l'image reconstruite pour chacune des ondes planes est une image de base angulée. Les circuits d'émission élémentaires transmettent une impulsion électronique avec déphasage aux transducteurs du réseau de sorte l'onde plane soit émise avec un angle différent par rapport à la direction de l'axe A. La sommation cohérente de toutes les images de base angulées permet de créer une focalisation synthétique et permet de reconstruire une image composée de meilleure qualité avec une cadence d'imagerie élevée. Sur la figure 5A sont représentées une onde plane à 0° et deux ondes planes angulées.

Selon un autre mode de réalisation et en référence à la figure 5B, les circuits d'émission élémentaires sont programmés pour ajouter un retard sur les transducteurs élémentaires de manière à émettre une onde ultrasonore divergente présentant un front d'onde convexe permettant d'insonifier un champ plus large que celui de l'onde plane. Les circuits d'émission élémentaires sont programmés par le système de contrôle 140 de manière à envoyer un retard aux transducteurs. La loi de retard appliquée aux transducteurs élémentaires 102 est inversée par rapport à l'imagerie focalisée. En d'autres termes, les transducteurs élémentaires situés au centre vont tirer en premier et les transducteurs situés à la périphérie vont tirer en dernier. Cela revient à considérer une source virtuelle 101* positionnée derrière le réseau sur son axe A, du côté opposé au milieu à imager.

Comme dans le cas de l'onde plane, afin d'augmenter la qualité d'imagerie, les circuits électroniques d'émission élémentaires sont programmés par le système de contrôle 140 de sorte que l'onde divergente est émise selon des axes déviés par rapport à l'axe A du réseau de transducteur. Cela revient à déplacer la source virtuelle d'un côté de l'autre de l'axe A pour déplacer le centre de la courbure de l'onde divergente. Ainsi, le milieu est insonifié avec une succession d'ondes divergentes avec des orientations différentes, l'image reconstruite pour chacune des ondes divergentes est une image de base. La sommation cohérente de toutes les images de base formées permet de reconstruire une image finale composée de meilleure qualité.

Selon un autre mode de réalisation et en référence à la figure 5C, les circuits d'émission élémentaires sont programmés par le système de contrôle 140 de sorte que les transducteurs élémentaires périphériques ne sont pas activés comme dans le cas de la figure 4B et pour ajouter un retard sur les transducteurs élémentaires activés de manière à émettre une onde ultrasonore divergente présentant un front d'onde convexe.

Pour chaque signal transmis, des signaux rétrodiffusés peuvent être générés par le milieu en réponse au signal transmis. Les signaux rétrodiffusés sont reçus par tous les transducteurs du réseau, à savoir les M*N transducteurs du réseau qui génèrent des signaux électriques. Ces signaux sont reçus par le circuit de réception 130 et peuvent être traités selon une stratégie de formation de faisceaux pour former une image.

La figure 2 illustre plus en détail le circuit de réception 130 selon un mode de réalisation.

Le circuit de réception 130 comprend un ensemble de circuits de micro-formation de faisceaux 131 situés directement dans la sonde 100 qui permet de réduire le nombre de signaux de réponse à transmettre depuis la sonde 100 vers le système de contrôle 140 en transmettant au système de contrôle 140 des signaux provenant d'un ensemble de sous-ouvertures de réception non focalisée formées par des groupes transducteurs ou des transducteurs élémentaires choisis dans chaque groupe. En d'autres termes, dans la phase de réception, les circuits de micro-formation de faisceaux 131 créent un réseau virtuel de réception avec un nombre réduit de signaux à transmettre depuis la sonde 100 vers le système de contrôle 140.

Selon un mode de réalisation, le signal provenant du réseau de transducteurs virtuels de réception peut correspondre aux signaux rétrodiffusés reçus par tous les transducteurs formant les sous-ouvertures de réception comme l'illustrent les figures 6A-6C.

Selon un autre mode de réalisation, le signal provenant du réseau virtuel de réception peut correspondre aux signaux rétrodiffusés reçus par l'un des transducteurs élémentaires de chaque groupe, les transducteurs étant choisis selon un motif ordonné ou selon un motif aléatoire comme l'illustrent les figures 7A et 7B.

Le circuit de micro- formation de faisceaux 131 permet ainsi de décomposer le processus de la formation d'une image 3D en deux étapes, une première étape de formation partielle au niveau de la sonde d'un nombre de signaux électriques parmi les signaux électriques générés par l'ensemble des transducteurs du réseau de manière à obtenir un nombre de signaux à transmettre depuis la sonde vers le dispositif de commande et traitement 140 inférieur ou égal au nombre de voies disponibles sur le système de contrôle 140 et une seconde étape de reconstruction de l'image 3D dans le système de contrôle 140 à partir des signaux microformés provenant des sous-ouvertures de réception formées par des groupes de transducteurs élémentaires ou par des transducteurs élémentaires choisis parmi les transducteurs élémentaires du réseau . En d'autres termes, après un tir d'onde, l'onde ultrasonore rétrodiffusée est captée par chaque transducteur du réseau. Le signal ainsi capté par chaque transducteur provient de l'ensemble du milieu, puisque l'onde incidente est une onde plane ou divergente. Les signaux rétrodiffusés sont captés par les M*P transducteurs, par exemple par 9*9 transducteurs dans le cas de la figure 1. Le nombre des M*P signaux captés par les transducteurs sont traités dans la sonde de manière à fournir un nombre Q de signaux, Q étant inférieur à M*P, et au maximum égal au nombre de voies disponibles sur le système de contrôle140. Les Q signaux formés sont transmis sur les canaux de transmission de signaux du câble 5 vers le système de contrôle140 aux fins de traitement pour reconstruire une image 3D ou un volume correspondant à l'onde incidente. Selon un exemple de réalisation, un circuit de multiplexage temporel peut relier la sortie du circuit de réception 130 aux canaux de transmission de signaux du câble 5. Le multiplexage temporel (ou TDM, de l'anglais Time Division Multiplexing) consiste à extraire une portion temporelle de N parmi les Q signaux - N étant un entier supérieur à 2 et inférieur ou égal à Q - et à transmettre l'une après l'autre les N portions temporelles dans un même canal du câble 5. Ce multiplexage permet avantageusement de réduire le nombre de canaux composant le câble 5.

Grâce à l'utilisation de sous-ouvertures de réception ou l'utilisation d'un nombre de transducteurs élémentaires choisis dans la limite de voies disponibles, il est possible d'utiliser un grand nombre de transducteurs élémentaires pour réaliser une imagerie volumétrique 3D avec un grand champ de vision, sans augmenter la complexité de l'électronique de pilotage et sa dimension, le rendant compatible dans une utilisation clinique.

Par ailleurs, afin d'augmenter l'angle de réception des ondes rétrodiffusées par chaque groupe de transducteurs élémentaires tout en conservant une surface sensible équivalente à celle de l'ensemble des transducteurs de ce groupe et la résolution spatiale de l'image 3D, chaque groupe de transducteurs élémentaires émule une lentille acoustique virtuelle ou un élément transducteur virtuel présentant une surface de réception de forme incurvée convexe orientée vers la région d'intérêt ou milieu à imager. Plus précisément, le circuit de micro-formation de faisceaux 131 est programmé par le système de contrôle 140 de sorte que les transducteurs élémentaires du groupe sont contrôlés indépendamment afin d'émuler un transducteur virtuel présentant une surface de réception de forme incurvée convexe ou une lentille virtuelle apte à faire converger l'onde reçu en un point focal situé derrière le réseau du côté opposé au milieu à imager.

En référence à la figure 2, un circuit de micro-formation de faisceaux 131 est décrit ci-dessous selon un mode de réalisation.

Selon un exemple de réalisation, les transducteurs élémentaires du réseau sont répartis en groupes 107 formant ainsi chacun une sous-matrice de n*n transducteurs adjacents. Dans l'exemple de la figure 2, la sous-matrice est un carré et n est égal à 3. Chaque transducteur élémentaire 102 appartient à un et un seul groupe élémentaire 107. Le circuit de réception 130 comprend donc un circuit électronique de micro-formation de faisceaux 131 pour chaque groupe de transducteurs élémentaires. Dans l'exemple de la figure 2, le circuit de réception 130 comprend donc neuf circuits électroniques de micro-formation de faisceaux 131 associés chacun à un groupe de transducteurs élémentaires.

Le circuit de micro-formation de faisceaux 131 comprend un circuit élémentaire de réception et à retard 132 pour chaque transducteur élémentaire du groupe 107, soit n*n circuits élémentaires à retard, et un circuit élémentaire de micro-sommation 133 par groupe. Comme l'illustre la figure 2, ce circuit de micro-formation de faisceaux 131 est relié individuellement à chaque transducteur élémentaire du groupe par l'intermédiaire d'un commutateur émission-réception 103 et configuré pour fournir un signal analogique ou numérique correspondant à une somme de signaux de réponse des transducteurs élémentaires du groupe.

Chaque circuit élémentaire de réception et à retard 132 présente une borne d'entrée reliée au commutateur d'émission-réception 103 et une borne de sortie. Chaque circuit élémentaire de réception et à retard 132 est adapté à fournir sur sa borne de sortie un signal de réponse conditionné et retardé d'un délai de réception prédéfini.

Chaque circuit de micro-sommation 133 comporte une borne d'entrée par transducteur élémentaire du groupe associé, soit n*n bornes d'entrée, et une borne de sortie unique. Les bornes d'entrée du circuit de sommation sont reliées, aux bornes de sortie des circuits élémentaire de réception et à retard 132.

Le circuit de sommation 133 est adapté à fournir, sur sa borne de sortie, un signal correspondant à la somme des n*n signaux de réponse conditionnés et retardés appliqués sur ses bornes d'entrée.

Sur l'exemple de la figure 2, chaque groupe comprend une sous-matrice de 3*3 transducteurs élémentaires. Chaque circuit de micro-formation de faisceaux 131 comprend donc 9 circuits élémentaires de réception et à retard 132.

La figure 3 illustre plus en détail le circuit élémentaire de réception et à retard 132 de la figure 2 selon un mode de réalisation. De manière connue, le circuit électronique de micro-formation de faisceaux peut être un circuit intégré spécialisé, dit ASIC (acronyme de l'anglais "application-specific integrated circuit").

Sur l'exemple de la figure 3, les transducteurs élémentaires du réseau sont répartis selon Q groupes de transducteurs élémentaires. Chaque groupe comprend W transducteurs élémentaires.

Le circuit de réception et à retard 132 peut comprendre un amplificateur de réception 132a, un circuit d'ajustement temporel de gain 132b ou TGC, de l'anglais time gain compensation) et un circuit à retard 132c.

L'amplificateur de réception 132a peut être par exemple un amplificateur linéaire à faible bruit.

Le circuit d'ajustement temporel de gain 132b est configuré pour appliquer un gain analogique variable en fonction du temps au signal de réponse du transducteur élémentaire au cours de l'étape de réception.

Chaque circuit à retards 132c présente une borne d'entrée reliée à la borne de sortie du circuit d'ajustement temporel de gain et une borne de sortie reliée au circuit de sommation 133. Le circuit à retards 132c est adapté à fournir sur sa borne de sortie un signal correspondant au signal appliqué sur sa borne d'entrée retardé d'une durée prédéterminée. Les circuits à retard 132c au sein de chaque groupe 107 sont programmés par le système de contrôle 140 pour appliquer une loi de retard sur les transducteurs élémentaires du groupe de sorte que le groupe émule une lentille virtuelle apte à faire converger l'onde reçu en un point focal situé derrière le réseau du côté opposé au milieu à imager. Les circuits amplificateurs de réception 132a, les circuits d'ajustement temporel de gain 132b de chaque groupe 107 sont également programmés par le système de contrôle 140 de façon à équilibrer l'amplitude des signaux de chaque transducteur élémentaire du groupe, voire à désactiver la contribution d'un ou plusieurs transducteurs élémentaires du groupe comme ce sera détaillé après.

Les circuits de micro-formation de faisceaux 131 sont ainsi configurés pour former un ensemble de sous-ouvertures de réception non focalisées ou un ensemble de lentilles virtuelles. En d'autres termes, le système de contrôle 140 reçoit des signaux de réponse transmis par un nouveau réseau virtuel qui permet de réduire le nombre de voies tout en maintenant la sensibilité en fournissant des ouvertures de réception non focalisées.

A titre d'exemple, le circuit à retard 132c peut être basé sur l'utilisation de capacités commutées. Dans ce cadre, l'utilisation de délais analogiques convergents permet avantageusement de réduire les délais analogiques maximum nécessaires par sous-ouverture, et par voie de conséquence la complexité de l'ASIC de chaque circuit à retard 132c associé. En effet, les retards analogiques sont liés au nombre de condensateurs qui prennent de la place dans l'ASIC. Le fait de limiter les retards permet d'augmenter la compacité de l'ASIC ou d'ajouter de nouvelles fonctionnalités dans la sonde.

Selon un mode de réalisation, le circuit de micro-formation de faisceaux 131 peut comprendre en outre un convertisseur analogique/numérique (non représenté) en sortie du circuit de micro-formation de faisceaux 131. Dans ce cas, les signaux fournis par les circuits de micro-formation de faisceaux 131 sont transmis vers le système de contrôle 140 sous forme numérique.

Sur l'exemple de la figure 3, le circuit de micro-formation de faisceaux 131 comprend donc pour chaque groupe, 9*9 amplificateurs de réception 132a, 9*9 circuits d'ajustement temporel 132b, 9*9 circuits à retard 132c, et un circuit de sommation 133.

Les figures 6A à 6C illustrent schématiquement trois exemples de réseau de lentilles virtuelles 102* présentant une surface de réception virtuelle de forme incurvée convexe orientée vers le milieu à imager et ayant pour point focal placé à l'arrière de la matrice du côté opposé à la région à imager. De telles lentilles virtuelles peuvent permettre de diminuer la directivité en agissant comme une lentille virtuelle. Le point focal virtuel 107* situé derrière le réseau peut être ajusté en choisissant le rayon de courbure de la forme incurvée.

La figure 6A représente une vue de côté (a) de la matrice de la figure 2 et une vue (b) en perspective de cette matrice. Sur l'exemple de la figure 6A, l'application du retard sur les transducteurs élémentaires au sein de chaque groupe 107 permet d'obtenir un réseau épars de neuf lentilles virtuelles 102*. Une telle micro-formation de faisceaux permet de garder la sensibilité en utilisant l'ensemble des transducteurs en réception tout en réduisant le nombre de voies. Sur l'exemple de la figure 6A, les lentilles virtuelles 102* obtenues présentent une surface incurvée convexe ayant les rayons de courbure identiques et les orientations identiques.

La figure 6B illustre un autre exemple de formation de lentilles virtuelles 102* présentant une surface de réception convexe avec un rayon de courbure plus important que celui des lentilles virtuelles de la figure 6A. Comme sur l'exemple de la figure 6A, les lentilles virtuelles 102* obtenues présentent une surface incurvée convexe ayant les mêmes rayons de courbure et les mêmes orientations.

La figure 6C illustre un autre exemple de formation de lentilles virtuelles 102* qui présentent une distribution aléatoire en rayon de courbure et en orientation.

Les exemples de réalisation présentées sur les figures 6A à 6C permettent de collecter le signal rétrodiffusé dans un secteur angulaire plus large tout en conservant la sensibilité. L'amélioration de la directivité des sous-ouvertures en réception permet de diminuer l'amplitude des lobes de réseau, et améliorer donc la qualité d'image. La variation dynamique de la courbure des lentilles virtuelles dans le cas de la figure 6A et figure 6B permet d'optimiser la reconstruction d'image en fonction de la profondeur. La variation pseudo-aléatoires de la répartition des sources virtuelles résultant en un réseau de lentilles virtuelles de courbures et d'orientations différentes de la figure 6C permet également de réduire la périodicité du réseau en réception et donc les amplitudes des lobes de réseau associées.

Les signaux retardés de manière appropriées par chaque circuit à retard 132c sont ensuite sommés analogiquement par le circuit de micro-sommation 133 pour former un signal micro-formé.

Le signal micro-formé fourni par chaque circuit de micro-formation de faisceaux 131 est ensuite transmis via le câble 5 vers le système de contrôle 140. Ainsi, la micro-formation de faisceaux permet de réduire le nombre de signaux de réponse à transmettre tout en maintenant la sensibilité de la sonde. Chaque groupe de transducteurs fournit un signal micro-formé représentatif de la somme ou superposition des signaux analogiques appliquées sur les bornes d'entrée du circuit de micro-sommation.

Le système de contrôle 140 peut ensuite reconstruire un volume ou une image 3D en appliquant un traitement de retard et sommation aux signaux micro-formés provenant des groupes de transducteurs. En d'autres termes, le système de contrôle applique une macro-formation de faisceaux sur des signaux provenant d'un réseau virtuel composé de lentilles virtuelles qui sont des macro-éléments correspondant chacun à un groupe de transducteurs élémentaires. Le système de contrôle 140 peut comprendre, comme dans la sonde, un circuit de macro-formation de faisceaux comprenant un circuit de réception et à retard pour chaque groupe et un circuit de sommation. Le circuit de réception et à retard peut comprend par exemple un amplificateur de réception, de préférence à faible bruit (LNA), un circuit d'ajustement temporel de gain (TGC) et un circuit à retard.

Pour améliorer la résolution et le contraste de l'image, il est utile de transmettre les ondes ultrasonores non focalisées en série d'ondes ultrasonores non focalisées successives, les ondes ultrasonores non focalisées successives de chaque série ayant respectivement des directions de propagation différentes. Dans ce cas, chaque images 3D est synthétisée à partir des signaux acquis à partir de la série.

Les ondes ultrasonores successives de chaque série peuvent être obtenues en faisant varier la source virtuelle 101*, d'une onde à l'autre, faisant varier le front d'onde comme le montrent les figures 5A-5C. Chaque série peut comprendre par exemple par exemple 1 à 81 ondes ultrasonores successives de directions différentes, par exemple 3 à 25 ondes ultrasonores successives de directions différentes, par exemple par exemple 5 à 20 ondes ultrasonores successives de directions différentes, par exemple 10 à 20 ondes ultrasonores successives de directions différentes.

Après chaque émission d'ondes ultrasonores, des signaux rétrodiffusés, sont acquis par le réseau 2D. Ces données brutes sont utilisées pour générer une séquence d'images.

Après avoir reçu les signaux rétrodiffusés, une formation de faisceaux peut être appliquée par le système de contrôle 140 pour reconstruire l'image 3D pour chaque onde ultrasonore émise. Les images 3D sont ensuite composées de manière cohérente pour former une image 3D finale de haute qualité. Le principe de cette imagerie synthétique est connu et décrit par exemple dans :

Papadacci, C., Pernot, M., Couade, M., Fink, M. & Tanter, M. High-contrast ultrafast imaging of the heart. IEEE transactions on ultrasonics, ferroelectrics, and frequency control 61, 288-301, doi:10.1109/tuffc.2014.6722614 (2014*).*

Montaldo, G., Tanter, M., Bercoff, J., Benech, N., Fink, M., 2009. Coherent plane-wave compounding for very high frame rate ultrasonography and transient elastography. IEEE Trans. Ultrason. Ferroelectr. Freq. Control 56, 489-506. doi:10.1109/TUFFC.2009.1067*.*

Selon un mode de réalisation, le système de contrôle 140 est configuré pour programmer le circuit électronique d'émission 120 et le circuit électronique de réception 130 de manière à répéter l'étape d'émission pour émettre une série de N ondes ultrasonores non focalisées, planes ou divergentes ayant des directions de propagation différentes et l'étape de réception pour recevoir N signaux bruts afin de construire une série de N images 3D ou N volumes.

Selon un mode de réalisation, le système de contrôle 140 est configuré pour reprogrammer le circuit d'émission 120 de manière à modifier l'ouverture d'émission pour chaque tir. Ce procédé permet alors de réduire la périodicité des sommes cohérentes successives et ainsi améliorer la qualité de l'image reconstruite en réduisant par exemple la formation de lobe de réseaux.

Selon un mode de réalisation, le système de contrôle 140 est configuré pour reprogrammer le circuit de réception 130, et plus précisément les circuits de réception et à retard de chaque groupe pour chaque tir, de manière à modifier les sous-ouvertures de réception non focalisées formées par des groupes de transducteurs ou les transducteurs élémentaires choisis dans chaque groupe pour la réception.

Dans le cas de modification des sous-ouvertures de réception, le système de contrôle 140 est configuré pour reprogrammer les circuits électroniques de micro-formation de faisceaux de chaque groupe pour chaque tir de manière à modifier les retards appliqués sur les circuits de retard 132C pour modifier l'orientation de la surface de réception convexe et leur rayon de courbure comme l'illustrent les exemples des figures 6A-6C.

Le système de contrôle 140 est configuré pour ensuite sommer de manière cohérente les images 3D obtenues après chaque tir afin de générer une image finale de haute qualité de la région.

La présence des circuits de micro-formation de faisceaux 131 dans la sonde 100 permet avantageusement de limiter le nombre de signaux à transmettre vers le système de contrôle 140 lors d'une phase de réception d'une onde ultrasonore. Le nombre total de signaux à transmettre qui correspond au nombre de transducteurs de la matrice est divisé par le nombre de groupes de transducteurs.

L'intégralité des signaux électriques générés par les circuits de micro-formation 131 peut être transmise vers le système de contrôle 140 pour former l'image 3D tout en limitant la taille du câble 5, permettant de mettre en oeuvre l'imagerie ultrarapide.

Par ailleurs, la possibilité de pouvoir reprogrammer les circuits de micro-formation de faisceaux 131 par le système de contrôle 140 permet avantageusement d'ajuster l'orientation des lentilles virtuelles ainsi que leur rayon courbure en fonction des paramètres d'émission et de reconstruction de l'image 3D souhaitée.

Les figures 7A et 7B illustrent un autre mode de réalisation dans lequel le système de contrôle 140 est configuré pour programmer le circuit de réception 130 de la figure 3 de manière à activer ou adresser un nombre réduit de transducteurs du réseau afin de fournir un nombre de signaux qui correspond au nombre de voies disponibles sur le système de contrôle 140 de l'appareil 1, permettant ainsi de réduire le nombre de canaux de transmission entre la sonde 100 et le système de contrôle 140.

Dans ce mode de réalisation, les circuits de micro-formation de faisceaux 131 situés dans la sonde 100 sont utilisés pour choisir un transducteur élémentaire à activer en réception parmi les transducteurs élémentaires dans chaque groupe afin de réduire le nombre de signaux de réponse à transmettre depuis la sonde 100 vers le système de contrôle 140. En d'autres termes, dans la phase de réception, les circuits de réduction de micro-formation de faisceaux 131 créent un réseau virtuel de réception avec un nombre réduit de signaux à transmettre depuis la sonde 100 vers le système de contrôle 140. Le système de contrôle 140 reçoit donc un ensemble de signaux provenant du réseau virtuel de réception.

Les transducteurs sont choisis dans chaque groupe par les circuits de micro-formation de faisceaux 131 selon un motif d'adressage ordonné ou selon un motif aléatoire comme l'illustrent respectivement les figures 7A et 7B.

Le circuit micro-formation de faisceaux 131 de la figure 3 est programmé par le système de contrôle 140 de manière à adresser un transducteur dans chaque groupe. Plus précisément, les circuits de réception et à retard 132 sont programmés de manière à adresser individuellement chaque transducteur élémentaire du groupe, c'est-à-dire à l'activer ou à ne pas l'activer. A titre d'exemple, le circuit d'ajustement temporel de gain 132b du circuit de réception et à retard 132 peut être programmé afin d'appliquer un gain nul au signal de réponse du transducteur élémentaire au cours de l'étape de réception pour ne pas activer un transducteur élémentaire. Les circuits à retards 132c sont programmés de manière à ne pas appliquer de retards sur les transducteurs élémentaires.

Selon un exemple de réalisation et comme l'illustre la figure 7A, le circuit micro-formation de faisceaux 131 de chaque groupe est programmé par le système de contrôle pour adresser un transducteur dans chaque groupe pour recevoir les ondes rétrodiffusées par le milieu. Le transducteur élémentaire activé pour recevoir les ondes rétrodiffusées est choisi selon un motif d'adressage identique pour tous les groupes du réseau. Le motif d'adressage global du réseau est ordonné. Le nombre total de transducteurs actifs peut être inférieur ou égal au nombre de voies de transmission disponibles. Sur l'exemple illustré, les carrés blancs désignent les transducteurs non actifs et les carrés sombres les transducteurs actifs.

Selon un autre exemple de réalisation et comme l'illustre la figure 7B, le circuit micro-formation de faisceaux 131 de chaque groupe est programmé par le système de contrôle 140 pour adresser un transducteur élémentaire dans chaque groupe pour recevoir les ondes rétrodiffusées par le milieu. Le transducteur activé pour recevoir les ondes rétrodiffusées est différent d'un groupe à l'autre. Le motif d'adressage global du réseau est aléatoire.

Selon un mode de réalisation, le système de contrôle 140 est configuré pour reprogrammer le circuit de réception 130, et plus précisément les circuits de réception et à retard 132 de chaque groupe pour chaque tir, de manière à modifier les transducteurs élémentaires choisis dans chaque groupe pour la réception.

Selon un exemple de réalisation, le motif d'adressage est identique pour tous les groupes dans un même tir mais différent entre les tirs. En d'autres termes, pour chaque groupe, les circuits de réception et à retard 132 sont programmés pour changer le choix du transducteur à activer à chaque tir.

La figure 7A illustre une succession de l'état des transducteurs élémentaires correspondant à une succession de tirs dans le cas de l'exemple d'un motif ordonné d'adressage. Dans un premier tir noté T₁, le motif d'adressage est répété pour tous les groupes, à savoir le premier transducteur élémentaire 102₁₁ de chaque groupe est activé. Dans le tir suivant noté T₂, le motif d'adressage (non visible sur la figure 7A) est différent et le circuit de micro-formation de faisceaux est programmé de manière à activer par exemple le deuxième transducteur élémentaire 102₁₂. Ce motif d'adressage est le même pour tous les groupes. Dans le dernier tir noté T₉, le circuit de micro-formation de faisceaux est programmé de manière à activer par exemple le dernier transducteur élémentaire du groupe 102₃₃. Ce motif est répété pour tous les autres groupes de ce même tir.

La figure 7B illustre une succession de l'état des transducteurs élémentaires correspondant à une succession de tirs dans le cas de l'exemple d'un motif aléatoire ou pseudo-aléatoire d'adressage. Dans le premier tir, le motif d'adressage est différent pour tous les groupes, c'est-à-dire le transducteur élémentaire activé est différent pour tous les groupes dans un même tir. Par exemple, dans le premier groupe, c'est le transducteur noté 102₃₂ qui est activé tandis que dans le deuxième groupe, c'est le transducteur noté 102₁₂ qui est activé. Dans le tir suivant noté T₂, le motif d'adressage (non visible sur la figure 7B) est différent de celui du premier tir.

Par voie de conséquence, que ce soit dans le cas d'un motif ordonné ou d'un motif aléatoire, à chaque tir, les transducteurs activés en réception sont différents. Comme dans le cas du mode de réalisation illustré sur les figures 6A-6C, l'image 3D finale ensuite est construite à partir de l'addition cohérente d'une série d'images 3D générées à partir de la succession de tirs pour améliorer la qualité de l'image 3D.

La figure 8 illustre les principales étapes d'un procédé d'imagerie ultrarapide volumétrique mettant en œuvre l'appareil d'imagerie 1 de la figure 2 et de la figure 3.

Dans une étape de programmation d'une émission (a) (PROGRAM_EMI), le système de contrôle 140 programme le circuit d'émission 120 pour utiliser un sous-ensemble de transducteurs élémentaires adjacents ou l'ensemble des transducteurs élémentaires du réseau 101 pour transmettre des ondes ultrasonores divergentes en direction de la région à imager. Le circuit d'émission 120 est programmé de sorte à activer les transducteurs élémentaires formant une ouverture d'émission qui détermine l'angle d'ouverture de l'onde ultrasonore et adaptée pour émettre une onde ultrasonore non focalisées plane ou divergente en direction de la région à imager.

Sur l'exemple du réseau de la figure 4A, le circuit d'émission 120 est programmé pour activer les 9*9 transducteurs élémentaires pour l'émission d'une onde ultrasonore plane ou divergente. Sur l'exemple de la figure 4B, le circuit d'émission 120 est programmé pour activer une partie des 9*9 transducteurs élémentaires pour l'émission d'une onde ultrasonore plane ou divergente

Dans une étape de programmation d'une réception (b) (PROGRAM_REC), le système de contrôle 140 programme les circuits de micro-formation de faisceaux 131 associés à chaque groupe de transducteurs pour appliquer un traitement de retards et sommation de sorte que chaque groupe de transducteurs émule une lentille virtuelle formant une sous-ouverture de réception non focalisée ou pour choisir un transducteur élémentaire à activer en réception parmi les transducteurs élémentaires dans chaque groupe selon un motif ordonné ou aléatoire.

Selon un mode de réalisation, le système de contrôle 140 programme les circuits de micro-formation de faisceaux 131 associés à chaque groupe de transducteurs pour appliquer un traitement de retards et sommation de sorte que chaque groupe de transducteurs émule une lentille acoustique virtuelle présentant une surface de réception de forme incurvée convexe dont le point focal est situé à l'arrière du réseau du côté opposé au milieu à imager comme l'illustrent les figures 6A-6C.

Selon un autre mode de réalisation, le système de contrôle 140 programme les circuits de micro-formation de faisceaux 131 associés à chaque groupe de transducteurs de manière à sélectionner le transducteur élémentaire du groupe à activer afin d'obtenir une distribution de transducteurs élémentaires actifs souhaitée selon un motif prédéfini comme l'illustrent les exemples des figures 7A-7B.

Dans une étape d'émission d'ondes ultrasonores (c) (EMIS_US), une onde ultrasonore non focalisée est émise en direction du milieu à imager par le réseau de transducteurs élémentaires activés par le circuit d'émission programmé par le système de contrôle. A titre de variante les étapes de programmation d'une réception (b) (PROGRAM_REC) et d'émission (c) (EMIS_US) peuvent être effectuées dans l'ordre inverse ou de manière simultanée.

Dans une étape de réception (d) (RECEP_US), les signaux rétrodiffusés sont reçus par les transducteurs de chaque groupe qui forme une sous-ouverture de réception non focalisée ou une lentille virtuelle. Pour chaque sous-ouverture de réception non focalisée, les signaux électriques générées peuvent être par exemple amplifiés par l'amplificateur 132a et/ou atténués par le circuit TGC 132b. Dans le cas de l'émulation d'une lentille virtuelle, les circuits à retards 132c appliquent ensuite des retards sur les signaux électriques. Dans le cas d'une distribution de transducteurs élémentaires activés selon un motif prédéfini, les circuits à retards 132c n'appliquent pas de retards sur les signaux électriques. Enfin, les signaux retardés sont sommés par le circuit de sommation 133 pour fournir en sortie du circuit de micro-formation de faisceau 131 un seul signal analogique micro-formé.

Dans une étape de transmission (e) (TRANS_SIG), les signaux fournis par l'ensemble des groupes sont transmis depuis la sonde 100 vers le système de contrôle 140 via le câble 5.

Dans une étape de construction d'un volume ou d'une image 3D (f) (CONST_IMG_3D), les signaux fournis par les groupes de transducteurs sont de nouveau retardés et sommés dans le système de contrôle 140 pour construire une image 3D ou un volume.

Pour améliorer la résolution et le contraste de l'image, les étapes (a) à (f) sont répétées pour émettre par le réseau de transducteur successivement une série de N ondes ultrasonore non focalisées ayant des directions de propagation différentes et pour recevoir par le réseau de transducteurs une série de signaux rétrodiffusés à partir de ladite série de N ondes ultrasonores non focalisées successives.

A titre d'exemple, chaque série peut comprendre par exemple 1 à 81 ondes ultrasonores successives de directions différentes, par exemple 3 à 25 ondes ultrasonores successives de directions différentes, par exemple 5 à 20 ondes ultrasonores successives de directions différentes, par exemple 10 à 20 ondes ultrasonores successives de directions différentes.

Selon un mode de réalisation, pour chaque tir, le système de contrôle 140 reprogramme le circuit d'émission pour modifier l'ouverture d'émission formée dans les transducteurs du réseau en modifiant par exemple la taille de l'ouverture d'émission et/ou les délais d'émission appliqués sur les transducteurs élémentaires.

Selon un mode de réalisation, pour chaque tir, le système de contrôle 140 reprogramme le circuit d'émission 120 pour modifier les retards d'émission appliqués sur les transducteurs élémentaires de manière à modifier la direction de propagation de l'onde ultrasonore comme indiqué ci-dessus. Selon un autre mode de réalisation, les circuits d'émission élémentaires sont reprogrammés par le système de contrôle 140 de sorte que les transducteurs élémentaires périphériques ne sont pas activés comme dans le cas de la figure 4B de manière à modifier la dimension de l'ouverture d'émission.

Selon un mode de réalisation, pour chaque tir, le système de contrôle 140 reprogramme le circuit de réception et plus précisément les circuits de micro-formation de voie associés à chaque groupe de transducteurs pour modifier les sous-ouvertures de réception et les retards de réception pour modifier l'orientation de la surface de réception de la lentille virtuelle formée par chaque groupe comme l'illustrent les exemples des figures 6A-6C.

Selon un autre mode de réalisation, pour chaque tir, le système de contrôle 140 reprogramme le circuit de réception et plus précisément les circuits de micro-formation de voie associés à chaque groupe de transducteurs pour modifier le motif d'adressage comme l'illustrent les exemples des figures 7A et 7B.

Dans tous les cas, après chaque émission d'ondes ultrasonores avec une direction de propagation donnée, des échos rétrodiffusés sont acquis par le réseau 2D. Ces données brutes (aussi appelées données RF ou données radiofréquences) sont micro-formées tout d'abord dans la sonde 100 comme indiqué ci-dessus dans l'étape (d) puis de nouveau macro-formés dans le système d'imagerie 140 comme indiqué dans l'étape (f) pour générer une image 3D.

A titre d'exemple, après une série de 10 tirs, c'est-à-dire 10 ondes ultrasonores successives de directions de propagation différentes, 10 images 3D sont donc sont formés.

Dans une étape de construction d'image finale de haute qualité (g) (CONST_IMG_COM), le procédé comprend une étape de construction d'image composée au cours de laquelle les images volumétriques 3D sont composées de manière cohérente pour former une image finale 3D de haute qualité.

Les étapes (a) à (f) sont répétées avec une cadence élevée comprise entre 5000 Hz et 20 000 Hz. La haute cadence est notamment requise en imagerie microscopie de localisation ultrasonore (ULM), et plus généralement pour suivre le mouvement des tissus ou des fluides.

## Revendications

1. Appareil d'imagerie ultrasonore tridimensionnelle (1) d'une région d'intérêt du corps d'un être vivant, ledit appareil comprenant :
- une sonde d'imagerie ultrasonore (100) comportant une pluralité de transducteurs élémentaires (102) agencés en matrice selon des rangées et des colonnes formant un réseau (101), lesdits transducteurs élémentaires étant répartis en un ensemble de groupes de transducteurs élémentaires (107), un circuit électronique d'émission (120) et un circuit électronique de réception (130) reliés aux transducteurs élémentaires (102);
- un système de contrôle (140) configuré pour communiquer avec la sonde (100);
- ledit circuit électronique de réception (130) comprenant un circuit de micro-formation de faisceaux (131) pour chaque groupe de transducteurs élémentaires (107), ledit circuit de micro-formation de faisceaux (131) ayant une entrée reliée individuellement à chaque transducteur élémentaire (102) du groupe et une sortie reliée au système de contrôle (140), ledit circuit de micro-formation de faisceaux (131) étant adapté à fournir un unique signal généré à partir des signaux de réponse des transducteurs élémentaires du groupe;
- ledit système de contrôle (140) étant configuré pour programmer pour chaque tir:
(a) le circuit électronique d'émission (120) de manière à adresser un ensemble de transducteurs élémentaires adjacents parmi la pluralité de transducteurs élémentaires afin de former une ouverture d'émission et à appliquer une loi de retards aux transducteurs élémentaires dudit ensemble pour l'émission d'une onde ultrasonore non focalisée;
(b) chaque circuit électronique de micro-formation de faisceaux (131) de manière à adresser le groupe de transducteurs élémentaires associé ou un transducteur élémentaire du groupe afin de former une sous-ouverture de réception ayant un point de focalisation situé derrière le réseau (101), du côté opposé à la région d'intérêt à imager, adaptée pour la réception des ondes rétrodiffusées, de sorte qu'une pluralité de sous-ouvertures de réception est formée.

2. Appareil selon la revendication 1, dans lequel le système de contrôle (140) est configuré pour reprogrammer le circuit électronique d'émission (120) pour modifier l'ouverture d'émission et les délais d'émission pour l'émission d'une onde ultrasonore non focalisée à chaque tir.

3. Appareil selon la revendication 1 ou 2, dans lequel le système de contrôle (140) est configuré pour reprogrammer les circuits électroniques de micro-formations de faisceaux (131) pour modifier les sous-ouvertures de réception et les délais de réception pour la réception des ondes rétrodiffusées à chaque tir.

4. Appareil selon l'une des revendications 1 à 3, dans lequel le système de contrôle (140) est configuré pour programmer le circuit électronique d'émission (120) pour appliquer des retards d'émission sur les transducteurs de sorte que l'onde ultrasonore non focalisée émise est une onde plane ou divergente.

5. Appareil selon l'une des revendications 1 à 4, dans lequel chaque circuit de micro-formation de faisceaux (131) comprend un circuit de réception et à retard (132) pour chaque transducteur (102) du groupe (107) et un circuit de micro-sommation des signaux (133).

6. Appareil selon la revendication 5, dans lequel les circuits de réception et à retard (132) du groupe sont aptes à appliquer une loi de retards sur les transducteurs élémentaires du groupe de manière à former une lentille virtuelle (102*) dont le point de focalisation (107*) est situé derrière le réseau, du côté opposé à la région à imager.

7. Appareil selon la revendication 6, dans lequel le système de contrôle (140) est configuré pour programmer les circuits de réception et à retard (132) de sorte que les lentilles virtuelles (102*) formées ont des distances focales identiques.

8. Appareil selon la revendication 6 ou 7, dans lequel le système de contrôle (140) est configuré pour programmer les circuits de réception et à retard (132) de sorte qu'au moins deux lentilles virtuelles (102*) ont des distances focales différentes.

9. Appareil selon l'une des revendications 6 à 8, dans lequel le système de contrôle (140) est configuré pour programmer les circuits de réception et à retard (132) de sorte qu'au moins deux lentilles virtuelles (102*) ont des directions de focalisation différentes.

10. Appareil selon l'une des revendications 6 à 9, dans lequel le circuit de réception et à retard (132) comprend un amplificateur de réception (132a), un circuit d'ajustement temporel de gain (132b) et un circuit à retard (132c).

11. Procédé d'imagerie ultrasonore tridimensionnelle (3D) d'une région d'intérêt du corps d'un être vivant à l'aide d'un appareil d'imagerie ultrasonore tridimensionnelle selon l'une des revendications 1 à 10, ledit procédé comprenant pour chaque tir:
- (a) une étape de programmation au cours de laquelle le circuit électronique d'émission (120) est programmé (PROGRAM_EMI) pour adresser un ensemble de transducteurs élémentaires adjacents parmi la pluralité de transducteurs élémentaires afin de former une ouverture d'émission et à appliquer une loi de retards adaptée aux transducteurs élémentaires dudit ensemble pour l'émission d'une onde ultrasonore non focalisée ;
- (b) une étape de programmation au cours de laquelle chaque circuit électronique de micro-formation de faisceaux (131) est programmé pour adresser le groupe de transducteurs élémentaires associé ou au moins un transducteur élémentaire du groupe afin de former une sous-ouverture de réception ayant un point de focalisation situé derrière le réseau (101), du côté opposé à la région d'intérêt à imager, adaptée pour la réception des ondes rétrodiffusés (PROGRAM_REC), de sorte qu'une pluralité de sous-ouvertures de réception est formée;
- (c) une étape d'émission au cours de laquelle l'onde ultrasonore non focalisée est émise en direction de la région d'intérêt par ladite ouverture d'émission formée par ledit ensemble de transducteurs élémentaires adjacents adressés (EMI_US) ;
- (d) une étape de réception au cours de laquelle les ondes rétrodiffusées provenant de la région d'intérêt sont reçues par chaque sous-ouverture non focalisée formée dans la sonde (REC_US);
- (e) une étape de transmission de signaux au cours de laquelle le signal fourni par chaque circuit de micro-formation de faisceaux (131) est transmis vers le système de contrôle (140) (TRANS_SIG);
- (f) une étape de construction d'image 3D au cours de laquelle les signaux sont retardés et sommés dans le système de contrôle (140) pour construire une image volumétriques ultrasonore 3D correspondant à l'onde émise (CONST_IMG_COM).

12. Procédé selon la revendication 11, dans lequel les étapes (a) à (f) sont répétées pour émettre une série d'ondes ultrasonore non focalisée selon différentes directions de propagation et pour acquérir une série de signaux pour chaque onde émise, lesdits signaux permettant de former une série d'images volumétriques 3D et dans lequel le procédé comprend en outre une étape de construction d'une image composée au cours de laquelle lesdites images volumétriques 3D sont sommées de manière cohérente pour former une image composée finale.

13. Procédé selon la revendication 12, dans lequel le système de contrôle (140) reprogramme le circuit d'émission (120) pour modifier l'ouverture d'émission et les délais d'émission pour l'émission d'une onde ultrasonore non focalisée à chaque tir.

14. Procédé selon la revendication 12 ou 13, dans lequel le système de contrôle (140) reprogramme le circuit électronique de micro-formation de faisceaux (131) pour modifier les sous-ouvertures de réception et les délais de réception pour la réception des ondes rétrodiffusées à chaque tir.
